# EUROPEAN PATENT APPLICATION

(11) **EP 3 323 879 A1**
(43) Date of publication of application: **23.05.2018**
(21) Application number: 16836579.9
(22) Date of filing: 09.08.2016
(51) Int. Cl.: C12M 3/00

(54) **CULTURE DEVICE FOR TISSUE CELL**

(30) Priority: 18.08.2015 CN 201510505129
(71) Applicant: Chongqing Runze Pharmaceutical Co. Ltd., Chongqing 400042 (CN)
(72) Inventor: YE, Lei, Chongqing 400042 (CN)
(74) Representative: Inal, Aysegul Seda
(86) International application number: PCT/CN2016/094180
(87) International publication number: WO 2017/028715

(57) **Abstract**

The present invention provides a culture device for tissue cell, and the culture device for tissue cell includes a tissue cell culture body. The porous material of the tissue cell culture body is composed by pore cavities which are classified according to the material pore size and cavity walls which are formed around the pore cavity at all levels. And the lower level small pore cavities are provided on the cavity wall formed around the upper level large pore cavities. The pore cavities within the same level are in communication with each other. And the pore cavities at all levels are also be in communication with each other. This device is particularly benefit for cell culture, so that tissue cells can freely and normally grow in three-dimensional space.

## Description

### Technical field

The present invention relates to a culture device for tissue cell culturing, and more particularly to a culture device for tissue cell culturing which is beneficial for free and normal growth of cells in a three-dimensional space under a culture environment.

### Background

Tissue cell culture is a technique that is very useful and widely used in the fields of drug development, cell biology, toxicology, bioengineering and tissue engineering. A culture device for tissue cell generally includes a culture container (such as a culture flask, a culture dish, a culture tank, etc.). During the culture, a culture medium is added into the culture container. The culture medium can be liquid (i.e., culture fluid) or solid. Generally, only culture medium is required when the cells are suspension cells, but when the cells are adherent cells, if there is only culture medium in the culture container, the adherent cells can only be cultured by attaching to the container wall. In the conventional cell culture, a cell culture plate is added to the culture container, such as cell culture plate with 2, 4, 6, 24, 96 pores, cell culture is performed in cell culture plate. The growth modes of cells cultured in the container wall or culture plate and cells grown in the body are not the similar. In the real living body, tissue cells are grown in three-dimensional space environment, so in the current two-dimensional planar cell culture plate, the growth of the cells is limited by the plane environment. Cell activity, morphology and growth status have great changes compared with the body environment. And the cells are easily exposed to the fluid shear stress of the culture fluid, which will cause cell damage. Therefore, the three-dimensional cell culture is of great significance, three-dimensional cell culture can closely simulate the situation in the body compared with the two-dimensional culture, with significant advantages over two-dimensional culture, especially in animal cells is more meaningful, because most animal cells grow adherently.

For performing three-dimensional cell culture, the researchers designed a number of devices:
CN101245313A discloses "three-dimensional cell culture inserts and methods of manufacture, kits, and uses thereof'. It describes a three-dimensional cell culture insert made of non-degradable, non-cytotoxic polymeric material with a defined and regular three-dimensional porous structure and consisting of a component with a single porous sheet or a plurality of porous sheet members fixed together. The three-dimensional porous structure allows the cells to attach to both the outer and inner surfaces of the three-dimensional structure at the same time, and that 100% interconnected porous structure can allow nutrients and metabolites to be easily exchanged, which is beneficial to cell culture.
CN102864119B discloses "carrier for cell culturing and preparation method thereof'. It describes the use of a three-dimensional porous graphene support as a cell carrier into a culturing tank for cell culture. The porous graphene support contains a metal foam that simulates the in vivo growth environment for the cells, to realize three-dimensional cell culture, which is conducive to maintaining the cell growth state and activity, promoting cell growth.

After carefully analyzing the prior arts described above, the disadvantages are as following; when the number of cultured cells is gradually increased, it not only blocks the pores and forms a physiological dead space or nutrient enrichment area, rendering the nutrients or metabolites difficult to flow and hindering the cells migration and growth, but also makes the uneven growth of cells in the culture body, and poor growth conditions.

### Summary of the Invention

The purpose of the present invention is to overcome the deficiencies in the prior art and to provide a culture device for tissue cell that facilitates cells to grow freely and normally in three dimensional space.

The inventor believes that if it is possible to provide a culture device for cells with the ability to satisfy the structural features that the nutrient solution or the metabolite freely flows in the porous culture body even if the pores of the cell culture body become gradually smaller or blocked with the increasing number of cells during cell growth and reproduction, so as to ensure the normal cell growth conditions, and overcome the deficiencies of the prior art.

The object of the present invention is achieved by the following technical solutions:
A culture device for tissue cell, includes a tissue cell culture body. A porous material of the tissue cell culture body is composed of pore cavities which are classified according to the pore size of the material and cavity walls surrounding to form the pore cavities, and a lower level of small pore cavity is provided on the cavity walls surrounding to form an upper level of large pore cavity. The pore cavities within the same level are interconnected with each other, and the pore cavities at different levels can also be interconnected with each other. The pore cavities of the porous material are classified, and a lower level of small pore cavity is provided on the cavity walls of an upper level of large pore cavities, so that the nutrient solution or the metabolite freely flows in the tissue cell culture body even if the pores of the cell culture body become gradually smaller with the increasing number of cells during cell growth and reproduction, which facilitates free and normal growth of the cells in three-dimensional space.

Further, in the porous material, the cavity walls surrounding to form the large cavities are formed by uniformly filling with the lower level of the small pore cavities, densely filling is preferred.

In the culture device for tissue cell, the pore cavities of the porous material used as tissue cell culture body is classified based on the pore size of the pore cavities, the pore cavities are divided into at least two levels. When the pore cavities of the porous material are divided into three levels, a next lower level of small pore cavities are provided on the cavity walls of the upper level of large pore cavities, or two lower levels of the small pore cavities are provided on the cavity walls of the largest level of pore cavities. And the like, for the pore cavities of the porous material to be divided into more levels, a lower level of small pore cavities are provided on the cavity walls of each of the upper level of the pore cavities, or a plurality of lower level of the small pore cavities are provided on the cavity walls surrounding to form of the upper level of the large pore cavities, or any combination of the plurality of lower level of the small pore cavities are provided on the cavity walls surrounding to form the upper level of the large pore cavities.

The inventor also believes that when the pore cavities of the porous material are divided into three levels, it is better that the cavity wall surrounding to form the upper level pore cavity is uniformly filled with the next lower level of pore cavities. Such pore classification not only facilitates cell growth but also facilitates preparation.

In the culture device for tissue cell culture, among the lower level of the pore cavities, pore sizes of the lowest level of the pore cavities are smaller than an average diameter of cultured cells, and the pore sizes of the other levels of the pore cavities are at least larger than the average diameter of the cultured cells.

In the culture device for tissue cell culture, the pore sizes of the larger level of the pore cavities are at least twice or more of the average diameter of cultured cells.

In the culture device for tissue cell, the pore sizes of the smallest level of the pore cavities are smaller than the average diameter of cultured cells. The pore sizes of the largest level of the pore cavities are preferably at least more than twice of the average diameter of cultured cells.

The tissue cell culture body mentioned above in the culture device for tissue cell is particularly suitable for the use of medical implant.

The culture device for tissue cell further includes a culture dish in which a tissue cell culture body is placed, the tissue cell culture body is immobilized on the culture dish.

In the culture device for tissue cell, the tissue cell culture body is preferably made into a plate structure.

In the culture device for tissue cell, the plate thickness of the tissue cell culture body represented as the plate structure is not larger than 20 times of the average pore size of the largest level of the pore cavity of the porous material.

In the culture device for tissue cell, the culture dish is fixed on a vibration working table.

The advantages of the present invention are as follows:
1. The present invention provides a culture device for tissue cell that facilitates the free and normal growth of cells in three-dimensional space. A porous material is used as a tissue cell culture body. In particular, the pore cavities of the porous material are classified by pore size of the porous material, the cavity walls surrounding to form the pore cavities are properly designed, and the pore cavities of the same level are interconnected with each other and the pore cavities within respective levels are also interconnected with each other, which creates a suitable space for three-dimensional free and normal growth of the cells. The small cavities are used for the transmission and the flow of nutrient solution or metabolites, the pore cavities that communicate with each other, provided on the cavity walls, can ensure the flow of nutrients or metabolites to form a microcirculation system to meet growth requirements of the cell. Large pore cavities are used for cells adherent living, and the cells can move freely through the three-dimensional space between the large pore cavities. Especially, when the pores are divided into three levels, the pore cavities at smallest level are used for the transmission and flow of the nutrient solution or metabolite, the pore cavities at middle level are used for adherent living of cells, and the pore cavities at largest level are used for migration and flow of cells. The culture body provided by the present invention does not form physiological dead space or concentration dead space in the cell culture process, and thus, avoids cell growth disorder area or nutrient enrichment area. Even if the cells increase, the pore cavities become smaller or get blocked, it does not hinder any infiltration or permeation of the nutrition fluid or metabolites in the cell culture body, so as to ensure the smooth and normal cell growth, which is in accordance with the cell growth rules.
2. In the culture device for tissue cell according to the present invention, the pore cavities of the porous material are classified, and the pore levels and the size of the pore cavities of the levels are reasonably designed, so as to meet various growth environment conditions of various tissue cells. In particular, the pore sizes of the pore cavities at smaller level are smaller than the average diameter of the cultured cells, which is more conducive to the transmission and flow of nutrients or metabolites. The pore sizes of pore cavities at other levels are at least larger than the average diameter of the cultured cells to further ensure the demand of the cell lives. The pore sizes of the pore cavities at larger level are at least twice of the average diameter of the cultured cells, so as to meet the needs of cell lives, so that the cells can freely migrate and travel, and to ensure the cells smoothly enter into the culture body, to migrate and growth in culture body. While the pore sizes of the pore cavities at the lower level are smaller than the average diameter of the cultured cells. Because of the small pore size, capillary force is generated, which can promote the infiltration and flow of the culture solution and the metabolites can inhale the cells into the cell culture body and promote the migration of the tissue cells in the tissue cell culture body. Furthermore, because of the presence of capillary force, the rate of cell growth will also be increased.
3. The cell culture body uses a cell culture plate structure, which makes it easy for the adherent cells grow into the interior, to avoid cell obstruction in the surface, but also facilitates the removal of cells, so as to facilitate the culture fluid flow through the cell culture body.
4. The tissue cell culture body provided by the present invention is placed in a culture dish and provided with an external vibration source for the culture conditions through the vibration working table, so as to perform vertical vibration, horizontal vibration and vibration rotating along the vertical axis, and at least two of the three vibrations are the compound vibration, so that the culture fluid in the culture dish flow can be promoted, and the concentration of tissue cells, and the distribution of various components are uniformly distributed.

### Brief description of the drawings

The present invention will be further described with reference to the accompanying drawings and embodiments.
Figure 1 is a schematic view of the culture device for tissue cell of the present invention;
Figure 2 is an A-A cross-sectional view of Figure 1 (culture dish cover 8 is removed);
Figure 3 is a structural schematic view of the tissue cell culture body, wherein 3-1 is a front view, 3-2 is a left side view, 3-3 is a top view;
Figure 4 is a partial enlarged view of the part B shown in Figure 3;
Figure 5 is a sectional diagram taken along C-C in Figure 4.

### Detailed description of the invention

Specific embodiments of the present invention are described below with reference to the accompanying drawings. The embodiments are given based on the technical solutions of the present invention, and specific embodiments and specific operational procedures are given. However, the protection scope of the present invention is not limited to the following embodiments.

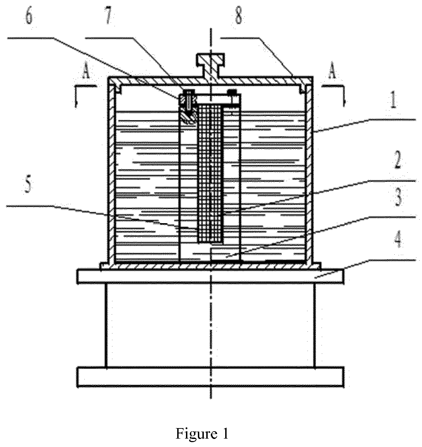

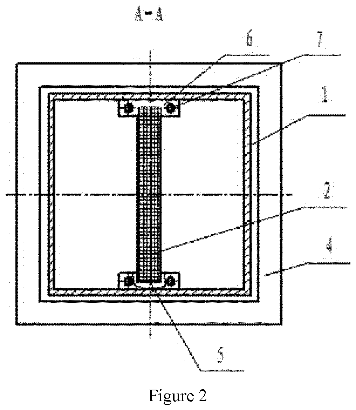

In Figures 1 and 2, 1 is referred to a culture dish, 2 is referred to a tissue cell culture body, 3 is referred to a fixing block configured to fix tissue cell culture body, which is fixed in the culture dish 1 by welding, 4 is referred to a vibration working table, and 5 is referred to a groove opened on the fixing block 3, configured for placing tissue cell culture body 2 into the fixing block , 6 is referred to a pressing block configured to fix the tissue cell culture body 2, 7 is referred to a screw, 8 is referred to a culture dish cover. The tissue cell culture body 2 is placed in the groove 5 of the fixing block 3, and the tissue cell culture body 2 is fixed on the fixing block 3 by means of the pressing block 6 and the screw 7, so as to fix the tissue cell culture body 2 in the culture dish 1, the culture dish 1 is fixed on the vibration working table 4 by common pressing plate and bolt fixing method, and the culture dish cover 8 covers the culture dish 1.

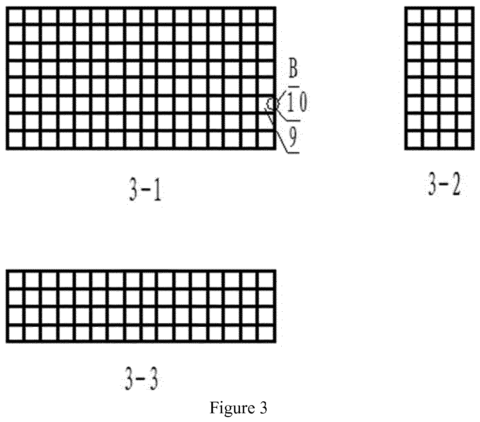

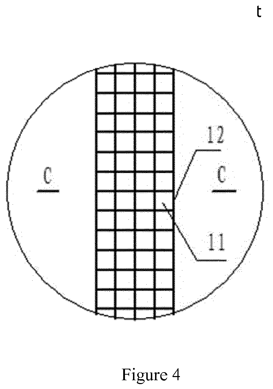

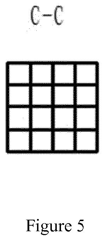

As shown in Figure 3, Figure 4, and Figure 5, 9 is a pore cavity of the largest level of the tissue cell culture body 2, and 10 is the cavity wall of the pore cavity 9 which is three-dimensionally interconnected. It can be seen from Figure 4, the cavity wall 10 of the pore cavity 9 is composed by a smaller pore cavity 11 (a next-level pore) and a cavity wall 12 surrounding the pore cavity 11. Referring to Figure 4, an enlarged view of the cavity wall 10 is shown, and Figure 5 shows a sectional diagram taken along C-C, the pore cavity 11 is also three-dimensionally interconnected, and the pore cavities of the largest level and the next-level are also three-dimensionally interconnected to each other.

By analogy, hierarchical porous structure with three or more levels of tissue cell culture body can be formed.

When culturing the cells, the cells are placed on the tissue cell culture body 2, and then the culture fluid is added to the culture dish 1, the culture dish cover 8 covers the culture dish 1, and the vibration working table 4 starts to vibrate the culture dish 1. After the culturing is completed, the cells on the tissue cell culture body 2 are taken out.

The embodiments of the present invention are given below in detail:

### Embodiment 1

Referring to drawings, a culture device for tissue cell comprises the tissue cell culture body 2. The tissue cell culture body 2 is fixed in the culture dish 1, and the culture dish 1 is fixed on the vibration table 4. The tissue cell culture body 2 is a porous tantalum material. The porous tantalum material is formed by the pore cavities which are classified into different levels according to the pore size of the material and the cavity walls surrounding to form the pore cavities of different levels, and the small pore cavities of a lower level are provided on the cavity walls surrounding to form the large pore cavity of an upper level. The pore cavities at the same level are interconnected with each other and the pore cavities between respective levels are also interconnected with each other.

Specifically, in this case, the porous tantalum material has two levels of the pore cavities, and the cavity walls of the large pore cavity of the upper level are formed by filling with the small pore cavities of the lower level, wherein the large pore cavities and the small pore cavities are uniformly distributed and are in communication with each other. The pore sizes of the large pore cavities of the upper level are 160µm - 460µm, the pore sizes of the small pore cavities of the lower level are 200nm - 500nm. The tissue cell culture body 2 is a plate material, wherein the thickness of tissue cell culture 2 is 5.5mm, which is 17 times of the average diameter of the large pore cavity of the upper level.

In the embodiment, smooth muscle tissue cells are cultured. During culturing, the smooth muscle tissue cells are placed in the tissue cell culture body 2, and MEM containing 12% serum is added into the culture dish 1. The temperature is controlled at 35 - 37 , the culture dish cover 5 is covered on the culture dish 1, and other specific operations are the same as the operations of the conventional cell culture technology. The vibration working table 4 is started, which makes the culture dish 1 vibrate, and the vibration adopts the vertical vibration mode, and the working frequency is 80Hz. After culturing for 3 days, the cells from the tissue cell culture body 2 were taken out and observed. The culture results showed that the cell bodies of the cultured cells were full and the growth state was good.

In this embodiment, the porous material is made according to the following methods:
(1) Material Preparation
   tantalum powders with a particle size of 1-10µm are used as raw material and the urea having a particle size of 300nm-600nm are used as a pore forming agent of the lowest level pores, and starch with a particle size of 300nm-600nm is used as binder The slurry is prepared by tantalum powder: urea: starch: distilled water mixed in a volume ratio of 1:1.5:1:7.
   The slurry is uniformly filled into polyester foam having a strut diameter of 260µm - 560µm by foam impregnation to form a green body and dry the green body. And then, the green body is crushed into mixed grains containing tantalum powder, pore forming agent, and polyester foam. The grain size of the mixed grains is 260µm - 560µm.
(2) The mixed grains and methylcellulose having a particle size of 260 m-560m are uniformly mixed based on a volume ratio of 2:1, and then placing the mixture into closed mold to press into compact green body.
(3) The compact green body is sintered in vacuum, and the sintered green body has a porous tantalum with two levels of pores after conventional follow-up treatment according to the tantalum process.

### Embodiment 2

The cell culture body 2 of the culture device for tissue cell in the embodiment uses a porous silicon dioxide material with three levels of the pore cavities, the cavity walls of the pore cavities of first level are formed by filling with the pore cavities of the second and third levels, and the pore cavities of the first level and the pore cavities of the second and third levels are uniformly distributed and interconnected with each other. Further, the pore cavities between respective levels are also interconnected with each other. The pore sizes of the large pore cavities of the first level are 1000µm - 1500µm, the pore sizes of the large pore cavities of the second level are 70µm - 90µm, and the pore sizes of the large pore cavities of the third level are 400nm - 700nm. The thickness of the tissue cell culture body 2 is 24mm, which is 19 times of the average pore size of the large pore cavity at the first level. The culture fluid is EAGLE containing 20 % calf serum. The cells to be cultured are epithelial cells. Other conditions are the same as that of embodiment 1.

The vibration working table is used in a vibration mode of rotating along the vertical axis, and the working frequency is 70Hz. The result of culture showed that the cell body was full and the growth state was good.

In the embodiment, the preparation method of the porous material is listed below:
(1) Material Preparation
   Silicon dioxide powder with a particle size of 1-10µm is used as raw material and the urea having a particle size of 500nm - 800nm is used as a pore forming agent for the lowest level pores (the third level), the ethyl cellulose having a particle size of 100µm - 150µm is used as a pore forming agent for the second level pores, and starch with a particle size of 500nm - 800nm is used as binder. A slurry is prepared by tantalum powder: urea: ethyl cellulose : starch : distilled water mixed in a volume ratio of 1 : 1.5 : 1 : 1 : 12.
(2) The slurry is uniformly filled into polyester foam having a pore size of 1100µm - 1700µm by foam impregnation to form a green body and conduct drying. And then, the green body is sintered in vacuum to obtain a porous silicon dioxide with three levels of pores.

### Embodiment 3

The cell culture body 2 of the culture device for tissue cell in the embodiment uses a porous niobium material with three levels of the pore cavities, wherein the cavity walls of the pore cavities of largest level (the first level) are formed by filling with the pore cavities of the middle level (the second level), the cavity walls of the pore cavities of second level are formed by filling with the pore cavities of the lowest level (the third level), and the pore cavities of first level are uniformly distributed and interconnected with each other, the pore cavities of the second level are uniformly distributed and interconnected with each other and the pore cavities of the third level are uniformly distributed and interconnected with each other. Further, the pore cavities at different levels are also interconnected with each other. The pore sizes of the pore cavities of the first level are 140µm - 600µm, the pore sizes of the pore cavities of the second level are 50µm - 90µm, and the pore sizes of the pore cavities of the third level are 300nm - 600nm. The thickness of the tissue cell culture body 2 is 17mm, which is 18 times of the average pore size of the pore cavities of the first level. The culture fluid is BME containing 18 % serum. The cells to be cultured are osteoblast. Other condition is the same as that of embodiment 1.

The vibration working table is used in a vibration mode that the horizontal vibration and the vibration of rotation along the vertical axis are carried out alternately, and the working frequency is 70Hz and 60 Hz, respectively. The result of culture showed that the growth state of the cells were good.

In this embodiment, the preparation method of the porous material is listed below:
(1) Material Preparation
   Niobium powder with a particle size of 1-10µm is used as raw material and the methyl cellulose having a particle size of 400nm - 700nm is used as a pore forming agent for the lowest level pores, the polystyrene having a particle size of 400µm - 700µm is used as binder. A slurry is prepared by tantalum powder: methyl cellulose : polystyrene : starch : distilled water according to a volume ratio of 1 : 2 : 1 : 7.5.
   The slurry is uniformly filled into polyester foam having a strut diameter of 60µm - 100µm and a pore size of 200µm - 400µm by foam impregnation to form a green body and drying. And then, the green body is crushed into mixed grains containing niobium powder, pore forming agent, and polyester foam. The grain particle size of the mixed particles is 60µm - 100µm.
(2) The mixed grains and ethyl cellulose having a particle size of 60µm -100µm are uniformly mixed in a volume ratio of 2: 1 and then, evenly pouring into polyester foam having a strut diameter of 250µm -750µm and a pore size of 400µm - 600µm which is three-dimensionally interconnected, after then, the treated polyester foam is put into a closed mold to press into a compact green body.
(3) The compact green body is sintered in vacuum, and the sintered body has a porous niobium with three levels of pores after conventional follow-up heat treatment to the niobium process.

### Embodiment 4

The tissue cell culture body 2 of the culture device for tissue cell in the embodiment uses a porous titanium material with four levels of the pore cavities, wherein the cavity walls of the pore cavities of largest level (i.e., the first level) are formed by filling with the pore cavities of the second level which are distributed uniformly and interconnected with each other, the cavity walls of the pore cavities of second level are formed by filling with the pore cavities of the third level and the fourth level which are distributed uniformly and interconnected with each other, further, the pore cavities at different levels are also interconnected with each other. The pore sizes of the pore cavities of the first level are 700µm - 1200µm, the pore sizes of the pore cavities of the second level are 200µm - 300µm, the pore sizes of the pore cavities of the third level are 400nm - 700nm, and the pore sizes of the pore cavities of at the fourth level are 10nm - 100nm. The thickness of the tissue cell culture body 2 is 17mm, which is 16 times of the average pore size of the pore cavities of the first level. The culture fluid is DMEM containing 10 % fetal bovine serum. The cells to be cultured are HUVEC cell. Other conditions are the same as that of embodiment 1.

The vibration working table is used in a vibration mode that the horizontal vibration and the vertical vibration are carried out alternately, wherein the horizontal vibration and the vertical vibration are vibrating for 2 minutes, respectively. And the working frequency is 50Hz and 70 Hz, respectively. The result of culture showed that the growth state of the cells was good.

In this embodiment, the preparation method of the porous material is listed as below:
(1) Material Preparation
   Titanium powder with a particle size of 1-10µm is used as raw material and the urea having a particle size of 5nm - 120nm is used as a pore forming agent for the lowest level pore cavities (the fourth level), the ethyl cellulose having a particle size of 500µm - 800µm is used as a pore forming agent for the third level pore cavities, and the starch having a particle size of 5µm - 120µm is used as binder. A slurry is prepared by tantalum powder: urea : ethyl cellulose : starch : distilled water according to a volume ratio of 1 : 1.5 : 1 : 1: 12.
   The slurry is uniformly filled into polyester foam having a strut diameter of 400µm - 600µm and a pore size of 400µm - 600µm by foam impregnation to form a green body and drying. And then, the green body is crushed into mixed grains containing titanium powder, pore forming agent, and polyester foam. The grain size of the mixed grains is 300µm - 400µm.
(2) The mixed grains and methyl cellulose having a grain size of 300µm -400µm are uniformly mixed in a volume ratio of 2: 1 and then, evenly pouring the mixture into polyester foam having a strut diameter of 800µm - 1300µm and a pore size of 1500µm - 2000µm which is three-dimensionally interconnected, after that, the treated polyester foam is put into closed mold to press into a compact green body.
(3) The compact green body is sintered in vacuum, and the sintered body has a porous titanium with four levels of pores after conventional follow-up heat treatment to the titanium process.

### Embodiment 5

The tissue cell culture body 2 of the culture device for tissue cell in the embodiment uses a porous CoNiCrMo alloy material with five levels of the pore cavities, wherein the cavity walls of the pore cavities of largest level (i.e., the first level) are formed by filling with the pore cavities of the second level and the third level which are distributed uniformly and interconnected with each other, the cavity walls of the pore cavities of the second level and the third level are formed by filling with the pore cavities of the fourth level and the fifth level which are distributed uniformly and are interconnected with each other. Further, the pore cavities of different levels are also interconnected with each other. The pore sizes of the pore cavities of the first level are 800µm - 1300µm, the pore sizes of the pore cavities of the second level are 100µm - 200µm, the pore sizes of the pore cavities of the third level are 30µm - 70µm, the pore sizes of the pore cavities of the fourth level are 500nm - 700nm, and the pore sizes of the pore cavities of the fifth level are 50nm - 200nm. The thickness of the tissue cell culture body 2 is 20mm, which is 18 times of the average pore size of the pore cavities of the first level. The culture fluid is DMEM containing 10 % fetal bovine serum and high glucose of double resistance. The cells to be cultured are HUVEC. Other condition is the same as that of embodiment 1.

The vibration working table is used in a horizontal vibration mode, and the working frequency is 70 Hz. The result of culture showed that the growth state of the cells was good.

In this embodiment, the porous material is made according to the following methods:
(1) Material Preparation
   CoNiCrMo alloy powder with a particle size of 1-10µm is used as raw material and the urea having a particle size of 60nm - 300nm is used as a pore forming agent for the lowest level pore cavities (the fifth level), the ethyl cellulose having a particle size of 600µm - 800µm is used as a pore forming agent for the fourth level pore cavities, and the starch having a particle size of 60µm - 300µm is used as binder. A slurry is prepared by CoNiCrMo alloy powder: urea : ethyl cellulose : starch : distilled water according to a volume ratio of 1 : 1.5 : 1 : 1: 12.
   The slurry is uniformly filled into polyester foam having a strut diameter of 40µm - 80µm and a pore size of 400µm - 600µm by foam impregnation to form a green body and conduct drying. And then, the green body is crushed into mixed grainss containing CoNiCrMo alloy powder material, pore forming agent, and polyester foam. The grain size of the mixed grains ranges from 40µm - 80µm.
(2) The mixed grains and methyl cellulose having a particle size of 200µm - 300µm are uniformly mixed at a volume ratio of 1: 1 and then, evenly pouring the mixture into polyester foam having a strut diameter of 900µm - 1400µm and a pore size of 1500µm - 2000µm which is three-dimensionally interconnected, then, the treated polyester foam is put into closed mold to press into a compact green body.
(3) The compact green body is sintered in vacuum, and the sintered body has a porous CoNiCrMo alloy with five levels of pores after conventional follow-up heat treatment to the CoNiCrMo alloy process.

## Claims

1. A culture device for tissue cell, comprising a tissue cell culture body, wherein a porous material of the tissue cell culture body is formed by pore cavities which are classified according to the material pore size and cavity walls which surrounding to form the pore cavity of different levels; and a lower level of small pore cavities are provided on the cavity walls surrounding to form an upper level of large pore cavities; the pore cavities within the same level are interconnected with each other, and the pore cavities of different levels are also interconnected with each other.

2. The culture device for tissue cell according to claim 1, **characterized in that**, in the porous material, the small pore cavities of the next lower level are provided on each of the upper levels of the cavity walls of the upper level of pore cavities, or small pore cavities of different lower levels are provided on the cavity walls surrounding to form the upper level of large pore cavities, or small pore cavities of different lower levels in arbitrary combination are provided on the cavity walls surrounding to form the upper level of the large pore cavities.

3. The culture device for tissue cell according to claim 1, **characterized in that**, when the pore cavities of the porous material are divided into three levels, a small pore cavity of the next lower level is provided on the cavity walls of the upper level of the large pore cavities, or a small pore cavities of the next two lower levels are provided on the cavity walls of the largest level of the pore cavities.

4. The culture device for tissue cell according to claim 1, 2, or 3, **characterized in that**, in the porous material, the cavity walls which surround to form the large cavity are formed by uniformly filling with the lower small pore cavities.

5. The culture device for tissue cell according to any one of claims 1 to 4, **characterized in that**, pore sizes of the lower level of the pore cavities is smaller than an average diameter of cultured cells, and the pore sizes of the pore cavities of other levels are at least larger than the average diameter of the cultured cells.

6. The culture device for tissue cell according to claim 5, **characterized in that**, the pore sizes of the larger level of the other levels of the pore cavities are at least twice of the average diameter of cultured cells.

7. The culture device for tissue cell according to any one of claims 1 to 6, **characterized in that**, the culture device for tissue cell further comprises a culture dish in which a tissue cell culture body is placed, the tissue cell culture body is immobilized on the culture dish.

8. The culture device for tissue cell according to claim 7, **characterized in that**, the tissue cell culture body is made into a plate structure.

9. The culture device for tissue cell according to claim 8, **characterized in that**, the plate thickness of the tissue cell culture body represented as the plate structure is not larger than 20 times of the average pore size of the largest level of pore cavities of the porous material.

10. The culture device for tissue cell according to any one of claims 7 to 9, **characterized in that**, the culture dish is fixed on a vibration working table.
